Europäisches Patentamt

European Patent Office

Office européen des brevets

(19)

(11) Publication number: **0 091 830**
**B1**

# (12) EUROPEAN PATENT SPECIFICATION

(45) Date of publication of patent specification: **12.08.87**

(21) Application number: **83302073.8**

(22) Date of filing: **13.04.83**

(51) Int. Cl.⁴: **B 01 D 5/00,** C 07 C 7/09,
F 25 J 3/06

(54) Separation of gas mixtures by partial condensation.

(30) Priority: **14.04.82 GB 8210820**

(43) Date of publication of application:
**19.10.83 Bulletin 83/42**

(45) Publication of the grant of the patent:
**12.08.87 Bulletin 87/33**

(84) Designated Contracting States:
**DE FR GB IT NL**

(56) References cited:
EP-A-0 050 468
EP-A-0 071 907
DE-A-2 924 179
DE-B-2 132 715
GB-A-1 181 049
US-A-4 110 996

(73) Proprietor: **COSTAIN PETROCARBON LIMITED**
**Petrocarbon House Sharston Road**
**Manchester M22 4TB (GB)**
(73) Proprietor: **British Gas Corporation**
**Rivermill House 152 Grosvenor Road**
**London SW1V 3JL (GB)**

(72) Inventor: **Duckett, Melvyn**
**13 Princes Drive**
**Marple Cheshire (GB)**
Inventor: **Ashton, Gregory Joseph**
**51 Parklands Way Poynton**
**Stockport Cheshire (GB)**
Inventor: **Salisbury, David Royson**
**39 Agincourt Street**
**Heywood Lancashire (GB)**

(74) Representative: **Cropp, John Anthony David**
**et al**
**MATHYS & SQUIRE 10 Fleet Street**
**London, EC4Y 1AY (GB)**

Courier Press, Leamington Spa, England.

**0 091 830**

**Description**

This invention is concerned with the separation of gas mixtures under pressure into two or more streams of differing composition by partial condensation at sub-ambient temperature, and in particular to such processes wherein a product stream derived from the condensate is to be recovered at a superatmospheric pressure not substantially below that at which the gas mixture is supplied.

A favoured procedure for the separation of a gas mixture is to cool it to a sub-ambient temperature in order to effect partial condensation and thereafter to separate the condensate from the uncondensed gas.

Where the cooling is effected by passing the feed gas mixture in indirect countercurrent heat exchange with one or more cold streams, it is of course essential for process operability that at all temperatures within the temperature range of the heat exchange the total enthalpy of the cold stream or streams exceed the enthalpy $H_F$ of the feed gas mixture to be cooled. To put it another way, if each of these enthalpies is plotted as ordinate against the temperature T as abscissa, the curve obtained for the cold stream or streams (the combined warming curve) must lie to the left of the curve obtained for the feed gas mixture (the cooling curve) throughout the whole of the temperature range.

If the sum of the enthalpies of the condensed and uncondensed gas streams obtained from the separation ($H_C$ and $H_G$, respectively) is plotted against the temperature T, the resultant curve will generally lie entirely to the right of the cooling curve within the entire temperature range if all the streams are at the same pressure. Such a process would be inoperable. As is well known, however, one means of rendering the process operable is to supply the feed gas mixture at a superatmospheric pressure and expand the condensate to a sufficient extent that the curve obtained from the sum of the enthalpies of the expanded condensate and the uncondensed gas falls entirely to the left of the cooling curve. The desired separation process may then be effected simply by employing the uncondensed gas and evaporating expanded condensate as the cooling streams in the heat exchanger to achieve the desired cooling and partial condensation of the feed gas mixture.

This solution is not very attractive, however, where it is desired to recover the evaporated condensate at a pressure not substantially below that at which the feed gas mixture is supplied, or indeed at any pressure which is above that to which it is necessary to expand the condensate.

An alternative is to render the process operable by means of a separate refrigerant stream. The enthalpy of this refrigerant stream must be such that the combined warming curve obtained by plotting the sum of the enthalpies of the condensate, uncondensed gas and refrigerant stream against temperature T lies entirely to the left of the cooling curve throughout the entire temperature range.

In many cases of gas separation by partial condensation, however, it is found that the difference $\Delta H$ between $H_F$ and $H_C + H_G$ is greater in one region of the temperature range than in another, warmer region. This means that when refrigeration is supplied by a separate refrigerant stream of conventional kind, the degree of refrigeration required to ensure that the combined warming curve lies to the left of the cooling curve throughout the entire temperature range is such that the curves become widely separated at the higher temperatures, resulting in a large temperature difference at the warm end. The system is thus over-refrigerated and hence inefficient.

In accordance with the present invention, this problem is overcome by employing a multi-component heat pump which will absorb heat within a first temperature region and deliver heat within a second, higher, temperature region within the range of temperature through which the feed gas mixture is to be cooled. By suitable choice of the composition of the multi-component heat pump medium and the pressures to which it is compressed and expanded in the heat pump cycle, the temperature regions within which it provides and removes heat may be adjusted to produce a combined warming curve having the desired shape to match closely that of the cooling curve throughout the entire temperature range. This modification to the shape of the cooling curve may be equated with providing the energy requirements of the separation process and is thus referred to in this specification. The heat pump may also, however, provide at least some, up to all, of the net refrigeration required for the process. In other words it may be such as not only to effect the desired correction to the shape of the combined warming curve but also to shift the curve along the abscissa axis so that it falls entirely to the left of the cooling curve. Alternatively, the refrigeration may be provided by a separate refrigeration cycle.

Thus, according to the present invention, there is provided a method of separating a gas mixture which comprises

cooling the gas mixture at superatmospheric pressure to sub-ambient temperature to partially condense it,

separating condensate from uncondensed gas,

without first subjecting the condensate to any significant Joule-Thomson expansion, returning the condensate in indirect counter-current heat exchange relationship with the feed gas mixture thereby evaporating and warming the condensate and providing a product stream, and

separately returning the uncondensed gas in indirect counter-current heat exchange relationship with the feed gas mixture to warm said uncondensed gas, and

wherein refrigeration requirements of the separation are satisfied by a separate refrigeration stream and the difference between the enthalpy of the feed gas mixture and the sum of the enthalpies of the returning streams comprising the condensate and uncondensed gas is greater in one region of the

2

temperature of the heat exchange between said feed gas mixture and said returning streams than in another, warmer, region,

characterised in that over-refrigeration in said warmer region is reduced by providing a heat pump wherein a multi-component gaseous medium is compressed, the compressed medium is thereafter condensed in indirect counter-current heat exchange relationship with said returning streams, the condensed medium is then expanded and evaporated in indirect counter-current heat exchange relationship with said feed gas mixture and condensed medium at a temperature range which is lower than that in which the medium is condensed, and the evaporated medium is returned for recompression.

By "without subjecting the condensate to any significant Joule-Thompson expansion" we mean that the condensate is not subjected to an expansion such as to provide any significant cold for the process; that is, an expansion of more than about 10% or 15% of the available pressure drop defined as the difference between the pressure at which the feed gas mixture is supplied for separation and atmospheric pressure. However expansion such as may occur through employment of e.g. flow control valves is not excluded.

By stating that the temperature range over which the expanded heat pump medium evaporates is lower than that over which the same medium is condensed, we mean that the upper value of the first-mentioned temperature range is below the upper value of the second-mentioned temperature range and likewise for the lower values. Thus the first-mentioned temperature range may fall entirely below the second or the ranges may overlap.

It is generally desirable to sub-cool the condensed heat exchange medium in order to reduce flash when it is expanded. The sub-cooling is conveniently effected by heat exchange with the separated streams and its expanded self and can be carried out in the same heat exchanger as the condensation.

The method provides a substantial saving in energy compared with the alternatives of effecting the process solely by expansion of the condensate or by a combination of employing expansion of the condensate to provide the refrigeration and employing a heat pump to provide the energy requirements; i.e. to adjust the shape of the combined warming curve to conform more closely with that of the cooling curve. Moreover in that embodiment of the present invention in which no separate refrigeration cycle is employed, the machinery requirement is reduced.

Where minor quantities of the uncondensed gas can be tolerated in the product obtained from the condensate, it has been found advantageous to inject a quantity of the uncondensed gas into the condensate prior to warming the latter in heat exchange with the incoming feed gas mixture. This has the effect of displacing the combined warming curve to the left in the lower temperature region and reduces the duty of the heat pump and the power consumed by it. While this benefit increases with increase in the amount injected, the injection counteracts the objective of the separation and therefore the amount injected will generally be relatively small. The optimum situation will depend upon the nature of the feed gas and the intended use of the separated condensate. By way of example, for the production of synthetic natural gas from hydrogen-containing gas streams such as those obtained from the gasification of solid hydrocarbon fuels or in the course of oil refining or petrochemical processes, the amount of uncondensed gas injected into the condensate typically will be about 2 to 3% by volume of the feed gas but may be as much as up to 8%.

In accordance with one embodiment of the invention, the heat pump is employed primarily or only to provide the energy for the separation and the refrigeration requirement is supplied by one or more separate refrigeration cycles. In this case, the heat pump will be substantially in balance; that is, so that there is no significant difference between the enthalpy $H_{CHP}$ of the compressed heat pump medium entering the warm end of the heat exchange system to be heat exchanged with the separated streams and the enthalpy $H_{EHP}$ of the expanded heat pump medium leaving said warm end of the heat exchange system after it has been evaporated by heat exchange with the feed gas mixture. The refrigeration requirements of the process are provided by any suitable refrigeration cycle or cycles.

In another embodiment, the heat pump provides not only the work for the separation but also at least part of the refrigeration. In this embodiment, the heat pump is designed to be out of balance so that $H_{EHP}$ is greater than $H_{CHP}$ and there is net refrigeration. The "unbalancing" may be achieved in known manner, e.g. by increasing the proportion of high boiling components in the multi-component heat pump cycle medium and if necessary the pressure to which the medium is compressed and/or by increasing the pressure drop to which the medium is submitted during expansion.

In yet another embodiment, the refrigeration may be provided by means of a vapour compression refrigeration cycle which employs as the cycle medium condensate obtained by partially condensing the compressed heat pump cycle medium prior to heat exchanging said compressed medium with the separated streams. To achieve this, the composition of the heat pump cycle medium will include at least one component which, after the medium has been compressed, can be condensed, e.g. by heat exchange with cooling water, prior to heat exchanging the compressed medium with the separated streams. The condensate is then separated from the uncondensed material prior to said heat exchange with the separated streams and employed in the refrigeration cycle, where it is first sub-cooled (usually) and then expanded and thereafter recombined with warming low pressure heat pump medium and evaporated in the warm section of the heat exchanger before returning to the suction side of the compressor.

As indicated above, the invention is particularly applicable to the separation of gas mixtures wherein in one region of the temperature range over which the gas mixture is cooled the difference ΔH between $H_F$

and $H_C + H_G$ is greater than in another, higher, region when the enthalpies are calculated for all the streams at the same pressure. Examples of such separation processes are those for the recovery of hydrogen-rich gases from hydrogen-containing gas streams such as those obtained by the gasification of coal or other solid hydrocarbon fuels or in the course of oil refining and petrochemical processes, especially hydrogen-containing streams which comprise mixtures of hydrogen with hydrocarbons containing not more than 80 mole % hydrogen, e.g. from 20 to 80 mole %, more particularly 35 to 70 mol %. The process of the invention is particularly suitable for the production of synthetic natural gas (SNG) from such streams.

The methods for determining the shapes of the warming curve and the combined cooling curve referred to above are well known and from this knowledge can be determined the nature of the heat pump cycle required to provide the work of separation.

For example, one convenient way of determining the parameters of the required heat pump cycle is to plot against temperature T the vertical gap $\Delta H$ between the cooling curve and the combined warming curve. The desired heat pump cycle is that in which the curve which is obtained by likewise plotting against temperature the enthalpy difference between the heat pump medium in the compressed form and the heat pump medium in the expanded form, corresponds as closely as possible to the aforementioned curve obtained for $\Delta H$ against T and lies above it at all points. The shape of the curve obtained for the heat pump cycle will be determined by the composition of the heat pump medium and the pressures to which it is compressed and expanded. The nature of the composition and the values for these pressures can be determined by simple experiment. In practice, the nature of the composition is selected on a trial and error basis but as a general rule it may be said that in the most widely applicable area of the invention, namely for processes for separating a hydrogen-rich gas from a feed gas stream containing hydrogen, the heat pump medium will usually comprise a mixture of light hydrocarbons (i.e. hydrocarbons containing 1 to 4 carbon atoms) and a minor proportion of nitrogen.

In many cases, it will be found that the hydrocarbon portion of the heat pump medium can be synthesised from the separated condensate and that the medium can be formed by adding one or more compounds, especially nitrogen, to a material having the same composition as the condensate or which has been derived from the condensate e.g. by distillation.

The amount of refrigeration provided and removed by the heat pump may be controlled by adjustment of the rate of circulation of the heat pump medium.

The invention is now described in more detail with reference to preferred embodiments thereof and with the aid of the accompanying drawings in which

Figure 1 is a flow sheet of an arrangement according to the invention in which an unbalanced heat pump provides all the refrigeration and the energy requirements for the separation

Figure 2 is a flow sheet of a similar arrangement but wherein at least some of the refrigeration is provided by a separate refrigeration cycle, and

Figure 3 is a flow sheet of an arrangement similar to that of Figure 2 but wherein the refrigeration is provided by a vapour compression refrigeration cycle wherein the refrigerant is divided from the medium employed in the heat pump cycle.

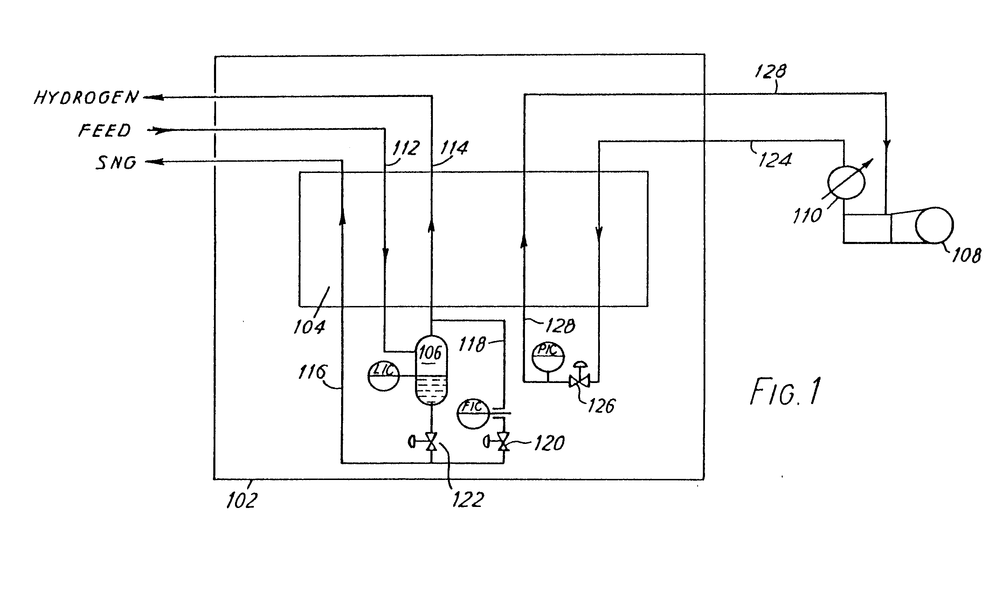

Referring to Figure 1, 102 is a cold box, 104 is a heat exchanger, 106 is a liquid/vapour separator, 108 is a compressor and 110 is a compressor after-cooler.

The feed gas mixture enters the cold box at superatmospheric pressure and ambient temperature through line 112, is cooled and partially condensed in heat exchanger 104 and the resulting liquid and vapour are separated in liquid/vapour separator 106, the uncondensed portion returning through heat exchanger 104 in line 114 and the condensate returning through line 116 in which it is warmed and evaporated by heat exchange in exchanger 104.

Prior to entering the cold end of the heat exchanger 104, a bleed stream 118 is withdrawn from the uncondensed gas and injected into the condensate in line 116. The rate of flow in line 118 is controlled by flow control valve 120 and that of condensate from the separator is controlled by level control valve 122.

The refrigeration and energy requirements of the process are provided by a heat pump in which a multi-component heat pump medium of appropriate composition is compressed to a first pressure in compressor 108 and, after the heat of compression has been rejected to cooling water in cooler 110, is passed via line 124 to enter the warm end of heat exchanger 104 wherein it is cooled and condensed over a first temperature range in heat exchange with the streams in lines 114, 116 and 128. The condensed medium is recovered from the cold end of the exchanger, expanded to a lower pressure through expansion valve 126 and thereafter returned via line 128 to enter the cold end of the exchanger wherein it is evaporated in heat exchange with the streams in lines 112 and 124 and over a second temperature range which is lower than the temperature range at which it was condensed. The heat pump provides refrigeration in the colder region of the heat exchanger and removes refrigeration from the warmer region, and can thus be arranged to ensure that the warming curve is sufficiently far to the left of the cooling curve for operability over the whole temperature range of the heat exchange system without unduly widening the gap at the higher temperatures of the system.

In order to provide the refrigeration requirements of the process, the composition of the heat pump medium and the pressure drop to which it is submitted on expansion through valve 126 are chosen such that the heat pump is unbalanced and produces the desired amount of net refrigeration.

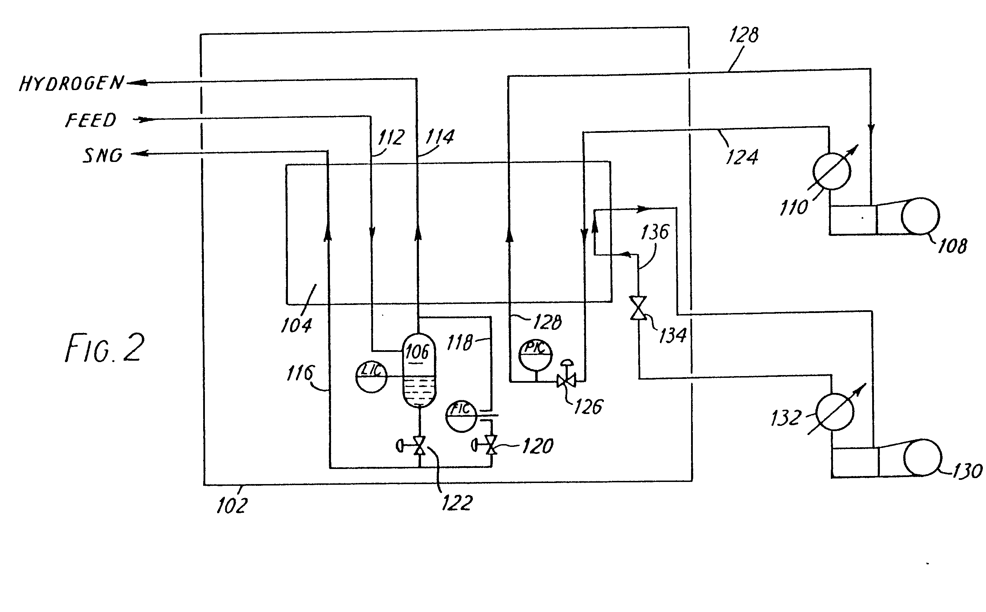

In the alternative arrangement illustrated in Figure 2, the features common with those of the

**0 091 830**

arrangement of Figure 1 have the same reference numerals, but there is a difference in that the composition of the heat pump medium and the pressure drop across expansion valve 126 are chosen such that the heat pump is not intentionally out of balance. In this arrangement, the refrigeration requirement for the process is satisfied by a separate refrigeration cycle which, in the embodiment illustrated, is a conventional vapour compression refrigeration cycle in which refrigerant is compressed in compressor 130, and, after the heat of compression has been removed in compressor after-cooler 132, expanded through expansion valve 134 and thereafter passed in line 136 through an additional passageway in heat exchanger 104, where it gives up its cold, and returned to the inlet of the compressor.

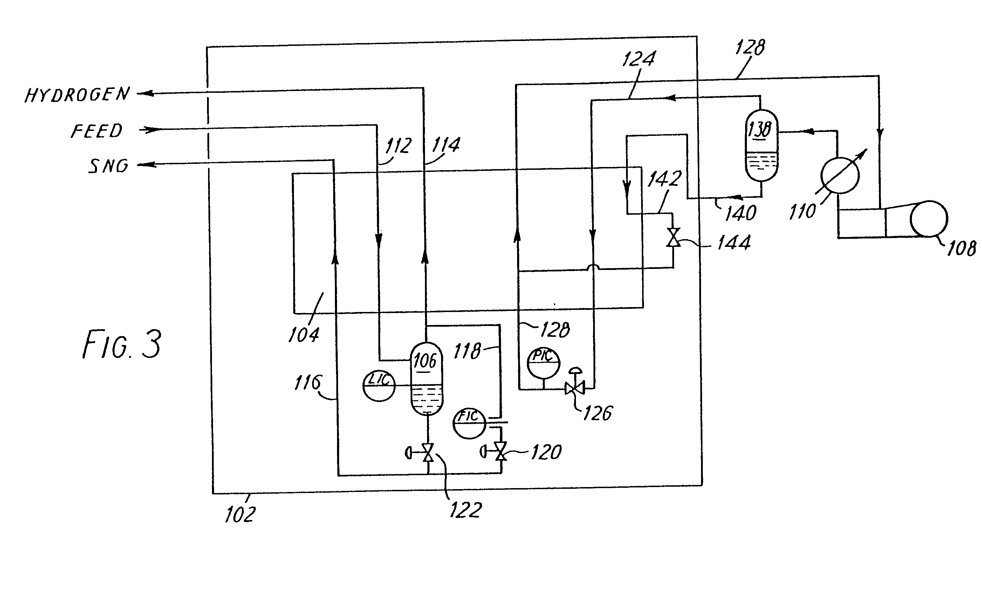

An alternative to the arrangement illustrated in Figure 2 is illustrated in Figure 3 wherein the features common with those of the arrangement of Figure 2 are again given the same reference numerals. In this arrangement, refrigeration for the process is provided by a vapour compression refrigeration cycle the refrigerant for which is provided by arranging for the heat pump medium to have a composition such that it is partially condensed on cooling in the compressor after-cooler 110. The mixture of vapour and liquid so obtained is then passed to vapour/liquid separator 138 and the uncondensed vapour is removed in line 124 to form the compressed heat pump medium which is condensed in heat exchanger 104 as in the arrangement of Figure 2. The condensate, which is recovered in line 140, forms the refrigerant for the vapour compression refrigeration cycle. It is sub-cooled in an extra passageway in heat exchanger 104, removed from the heat exchanger at an intermediate position through line 142, expanded through expansion valve 144 and thereafter injected into line 128, which carries the evaporating low pressure heat pump medium through the heat exchanger 104, at another intermediate point in the heat exchanger, whereby it is evaporated in the heat exchanger to satisfy the refrigeration requirements of the process.

Modifications of the arrangements illustrated in Figures 1 to 3 will be apparent to those skilled in the art. For example, heat exchanger 104 may be replaced by two or more heat exchangers which may be in parallel and/or in series. Either or both of compressors 108 and 130 may be multi-stage compressors. The single stage partial condensation of the feed gas mixture entering in line 112 may be replaced by a multi-stage process e.g. with separation of condensate after each stage. The partial condensation of the compressed medium recovered from the compressor 108 in the arrangement illustrated in Figure 3 may be effected in part in the compressor after-cooler 110 and in part in at least one further heat exchanger or alternatively entirely in a further heat exchanger or exchangers. The refrigeration requirements may be provided in part by the heat pump and in part by a separate refrigeration cycle and/or by a refrigeration cycle in which the refrigerant is obtained by partial condensation of the heat pump medium prior to heat exchanging same with the returning streams in the heat exchanger. Moreover, the process can be achieved, although less efficiently, without the injection of the bleed stream 118 into the condensate line 116.

The invention is now illustrated by the following Examples in each of which a feed stream having the composition and flow rate given below is separated into a synthetic natural gas (SNG) stream and a residual hydrogen stream, each having the composition and flow rate indicated.

| Composition | Feed entering in line 112 mol % | Hydrogen recovered from plant in line 114 mol % | SNG recovered from plant in line 116 mol % |
|---|---|---|---|
| $H_2$ | 56.03 | 95.08 | 10.00 |
| $N_2$ | 0.06 | 0.06 | 0.06 |
| CO | 0.84 | 0.67 | 1.04 |
| Ar | 0.09 | 0.05 | 0.14 |
| $CH_4$ | 34.94 | 4.14 | 71.25 |
| $C_2H_4$ | 0.12 | — | 0.26 |
| $C_2H_6$ | 7.92 | — | 17.25 |
| Flow Rate kmol/hr | 10544 | 5704.4 | 4839.6 |

The flow in line 118 equals about 3% of the flow rate of the Feed.

Example 1

Using the arrangement illustrated in Figure 1, the feed gas mixture in line 112 enters the cold box 102 at 58.6 bar absolute and is cooled in heat exchanger 104 from 303 K to 115 K. The condensate recovered from

5

vapour/liquid separator 106 under level control, and containing uncondensed gas injected through line 118, is evaporated in the heat exchanger and recovered at 57.6 bar absolute and 300 K. The vapour in line 114 is also recovered from the heat exchanger at 57.6 bar absolute and 300 K.

To provide the energy and refrigeration requirements of the process, a heat pump medium having the following molar composition: nitrogen 12%, methane 44%, ethylene 39%, isobutane 5%, is circulated at a rate of 2333 kmol/hr through compressor 108, compressor after-cooler 110, line 124, expansion valve 126 and line 128. After leaving the compressor 108 at 50 bar, the heat of compression is removed in after-cooler 110 and the medium enters the warm end of heat exchanger 104 in line 124 wherein it is cooled, condensed and sub-cooled to 115 K. It is then expanded to 2.0 bar absolute and 108.5 K in valve 126 and returned to the heat exchanger in line 128 wherein it is evaporated and warmed to 300 K before being returned to the suction side of the compressor at a pressure of 1.8 bar absolute.

The power consumption of the compressor is 8010 kW.

By way of comparison, if the same feed gas is separated into the same products but with the energy and refrigeration requirements of the process being supplied by expansion of the condensate in line 116 instead of using a heat pump, it is necessary to expand the condensate to 3 bar absolute and the power required to recompress the SNG to the same recovery pressure of 57.6 bar absolute is 14500 kW. The process of the invention therefore provides an energy saving of 45%.

Example 2

The pressures and temperatures of the incoming feed gas mixture, the recovered SNG and the recovered hydrogen stream, and the temperature to which the feed gas mixture is cooled in the heat exchanger, are the same as in Example 1. In this Example, however, the arrangement of Figure 2 is employed; that is, the heat pump cycle is arranged to be more nearly in balance than in the arrangement of Figure 1 and a significant part of the refrigeration requirements are provided by a separate refrigeration cycle.

To achieve this, the composition of the heat pump medium is adjusted to 12% nitrogen, 41% methane, 42% ethylene and 5% isobutane (molar) and is circulated at a rate of 3045 kmol/hr. It is compressed to 30.0 bar absolute in compressor 108 and, after removal of the heat of compression, is condensed in heat exchanger 104 and sub-cooled to 115 K. The condensate is then expanded to 3.0 bar absolute and 110 K in expansion valve 126 and returned to the heat exchanger where it is evaporated and warmed to 300 K before being returned to the suction side of the compressor.

To provide the refrigeration requirements, refrigerant R22 is supplied in line 136 to heat exchanger 104 at a pressure of 2.16 bar absolute, a temperature of 250 K and a flow rate of 106 kmol/hr.

The power consumptions of the heat pump compressor and refrigerator compressor are 7477 and 161 kW, respectively, a total of 7638 kW. This arrangement is thus even more economical in power than that of Example 1.

By way of comparison, if the process is repeated but with the refrigeration requirements provided instead by expansion of the condensate recovered from the vapour/liquid separator 106 instead of by the separate refrigeration cycle, it would be necessary to expand the condensate to 19.5 bar absolute. In order to adjust for the consequential change in distribution of heat and cold requirements in the heat exchanger 104, it is necessary to adjust the composition and conditions of the heat pump cycle. The altered conditions are satisfied by a heat pump medium composition of 15% nitrogen, 65% methane and 20% ethylene (molar), a flow rate of 1061.4 kmol/hr and pressures of 20 bar and 1.5 bar, requiring a heat pump compressor power consumption of 2830 kW. This power consumption is reduced because a substantial part of the energy of separation is also supplied by the expansion of the condensate.

The power requirements for re-compressing the expanded condensate from 19.5 bar to 57.6 bar absolute are 5440 kW, making a total of 8270 kW, an increase of about 8%.

If Example 1 or Example 2 is repeated but without the injection of the bleed stream in line 118 into the condensate in line 116, a lower but still significant power saving can be obtained by means of the processes described therein, compared with the conventional arrangement whereby the refrigeration and energy requirements are provided by expanding the condensate which thereafter has to be recompressed.

Example 3

The pressure and temperature of the incoming feed gas mixture and the temperature to which the feed gas mixture is cooled in the heat exchanger 104 are the same as in Example 1. In this Example, however, the arrangement of Figure 3 is employed.

A stream having a composition of 12% nitrogen, 41% methane, 30% ethylene and 17% isobutane and circulating at the rate of 3600 kmol/hr and which has been compressed to 40 bar absolute in compressor 108 is cooled in after-cooler 110 to 303 K and thereby partially condensed to produce 152 kmol/hr of liquid. This liquid is separated in separator 138 and recovered in line 140, passed to heat exchanger 104 where it is sub-cooled and recovered therefrom at an intermediate point in line 142 at 250 K. It is then expanded to 3.0 bar absolute and 230 K in expansion valve 144 and returned to the exchanger at another intermediate point to provide refrigeration for the process.

The uncondensed vapour recovered from separator 138 is condensed and sub-cooled in line 124 in heat exchanger 104 to 115 K, expanded to 3.0 bar absolute and 110.6 K and returned in line 128 to the heat

6

0 091 830

exchanger wherein it is warmed to 235 K, combined with the expanded fluid from the refrigeration circuit and the mixture recovered as a gas from the warm end of the exchanger at 295 K. The hydrogen and SNG streams are also both recovered at 295 K.

The power consumption of compressor 108 in this case is 9778 kW.

In all the above Examples, it is possible to replace the ethylene in the heat pump medium by ethane.

**Claims**

1. A method of separating a gas mixture which comprises:—

cooling the gas mixture at superatmospheric pressure to sub-ambient temperature to partially condense it,

separating condensate from uncondensed gas,

without first subjecting the condensate to any significant Joule-Thomson expansion, returning the condensate in indirect counter-current heat exchange relationship with the feed gas mixture thereby evaporating and warming the condensate and providing a product stream, and

separately returning the uncondensed gas in indirect counter-current heat exchange relationship with the feed gas mixture to warm said uncondensed gas, and

wherein refrigeration requirements of the separation are satisfied by a separate refrigeration stream and the difference between the enthalpy of the feed gas mixture and the sum of the enthalpies of the returning streams comprising the condensate and uncondensed gas is greater in one region of the temperature of the heat exchange between said feed gas mixture and said returning streams than in another, warmer, region,

characterised in that over-refrigeration in said warmer region is reduced by providing a heat pump wherein a multi-component gaseous medium is compressed, the compressed medium is thereafter condensed in indirect counter-current heat exchange relationship with said returning streams, the condensed medium is then expanded and evaporated in indirect counter-current heat exchange relationship with said feed gas mixture and condensed medium at a temperature range which is lower than that in which the medium is condensed, and the evaporated medium is returned for recompression.

2. A process as claimed in claim 1 wherein the heat pump also supplies refrigeration for the process.

3. A process as claimed in claim 1 wherein the refrigeration is supplied by means of a vapour compression refrigeration cycle wherein the cycle medium comprises condensate obtained by partially condensing the compressed heat pump medium prior to passing said compressed medium in indirect heat exchange relationship with said separated streams.

4. A process as claimed in any one of the preceding claims in which the duty of the heat pump is reduced by injecting a portion of the uncondensed gas into the separated condensate prior to warming the latter by indirect heat exchange with incoming feed gas mixture.

5. A process as claimed in any one of the preceding claims in which the condensed heat pump medium is sub-cooled prior to expansion.

6. A process as claimed in any one of the preceding claims in which the gas mixture comprises a mixture of hydrocarbons and hydrogen and contains 20 to 80 mol % hydrogen.

7. A process as claimed in any one of the preceding claims for the production of synthetic natural gas from a hydrogen-containing gas stream obtained by the gasification of a solid fuel or in the course of oil refining or a petrochemical process.

**Patentansprüche**

1. Verfahren zum Trennen eines Gasgemisches, bei dem

— das Gasgemisch bei überatmosphärischem Druck auf Unterumgebungstemperatur abgekühlt wird, um es teilweise zu kondensieren,
— das Kondensat von dem unkondensierten Gas abgetrennt wird,
— das Kondensat, ohne es zunächst einer signifikanten Joule-Thomson-Expansion zu unterwerfen, in indirekter Gegenstrom-Wärmetauscher-Beziehung zu dem zugeführten Gasgemisch zurückgeführt wird,

wodurch das Kondensat verdampft, sich erwärmt und einen Produktstrom liefert, und

— das unkondensierte Gas in indirekter Gegenstrom-Wärmetauscher-Beziehung mit dem zugeführten Gasgemisch getrennt zurückgeführt wird, um das unkondensierte Gas zu erwärmen, und
— wobei die Kälteerzeugungs-Anforderungen der Trennung durch einen getrennten Kälteerzeugungsstrom befriedigt werden und die Differenz zwischen der Enthalpy des zugeführten Gasgemisches und der Summe der Enthalpien der zurückkehrenden Ströme aus dem Kondensat und dem unkondensierten Gas in einem Temperaturbereich des Wärmetausches zwischen dem zugeführten Gasgemisch und den zurückkehrenden Strömen größer als in einem anderen, wärmeren Bereich ist,

dadurch gekennzeichnet, daß

7

# 0 091 830

— die über-Kälteerzeugung in dem wärmeren Bereich durch Vorsehen einer Wärmepumpe reduziert wird, in der ein aus mehreren Komponenten bestehendes, gasförmiges Medium komprimiert, das komprimierte Medium anschließend in indirekter Gegenstrom-Wärmetauscher-Beziehung zu den zurückkehrenden Strömen kondensiert, das kondensierte Medium dann expandiert und in indirekter Gegenstrom-Wärmetauscher-Beziehung zu dem zugeführten Gasgemisch und dem kondensierten Medium bei einem Temperaturbereich, der niedriger als der Bereich ist, in dem das Medium kondensiert wird, verdampft und das verdampfte Medium zur erneuten Kompression zurückgeführt wird.

2. Verfahren nach Anspruch 1, bei dem
— die Wärmepumpe auch die Kälteerzeugung für das Verfahren liefert.

3. Verfahren nach Anspruch 1, bei dem die Kälteerzeugung mittels eines Dampfkompressions-Kälteerzeugungszyklus geliefert wird, wobei das Zyklus-Medium Kondensat aufweist, das durch teilweise Kondensation des komprimierten Wärmepumpen-Mediums vor der Führung des komprimierten Mediums in indirekter Wärmetauscher-Beziehung mit den getrennten Strömen erhalten wird.

4. Verfahren nach einem der vorhergehenden Ansprüche, bei dem die Leistung der Wärmepumpe durch Injektion eines Teils des unkondensierten Gases in das abgetrennte Kondensat vor der Erwärmung des abgetrennten Kondensates durch indirekten Wärmetausch mit dem ankommenden, zugeführten Gasgemisch reduziert ist.

5. Verfahren nach einem der vorhergehenden Ansprüche, bei dem das kondensierte Wärmepumpen-Medium vor der Expansion unterkühlt ist.

6. Verfahren nach einem der vorhergehenden Ansprüche, bei dem das Gasgemisch ein Gemisch aus Kohlenwasserstoffen und Wasserstoff aufweist und 20 bis 80 Mol-% Wasserstoff enthält.

7. Verfahren nach einem der vorhergehenden Ansprüche für die Herstellung von Ersatzgas für Erdgas aus einem Wasserstoff enthaltenden Gasstrom, der durch Vergasung eines festen Brennstoffes oder im Verlaufe der Erdölverarbeitung oder bei einem petrochemischen Prozess erhalten wird.

**Revendications**

1. Procédé de séparation d'un mélange gazeux qui comprend:
le refroidissement du mélange gazeux à pression super-atmosphérique à une température inférieure à la température ambiante pour le condenser partiellement,
la séparation du condensat du gaz non-condensé,
sans soumettre en premier le condensat jusqu'à une détente Joule-Thompson importante, puis recycler le condensat à contre-courant par échange de chaleur indirect avec le mélange gazeux d'alimentation et ainsi évaporer et chauffer le condensat et fournir un courant de produit et recycler séparément le gaz non-condensé à contre-courant par échange de chaleur indirect avec le mélange gazeux d'alimentation pour chauffer ledit gaz non-condensé et
dans lequel les exigences de réfrigération de la séparation sont remplies par un courant de réfrigération séparé et la différence entre l'enthalpie du mélange gazeux d'alimentation et la somme des enthalpies des courants de recyclage comprenant le gaz condensé et non-condensé est plus grande dans une zone de la température d'échange de chaleur entre ledit mélange gazeux d'alimentation et lesdits courants de recyclage que dans une autre zone de température plus chaude,
caractérisée en ce que la surréfrigération dans ladite zone est réduite en prévoyant une pompe de chaleur dans laquelle est compressé un mélange gazeux à composant multiple, le milieu compressé est ensuite condensé à contre-courant par échange de chaleur indirect avec lesdits courants de recyclage, puis le milieu condensé est détendu et évaporé à contre-courant par échange de chaleur indirect avec le mélange gazeux d'alimentation et le milieu condensé à un domaine de température qui est inférieur à celui dans lequel le milieu est condensé, et le milieu évaporé est recyclé pour recompression.

2. Procédé selon la revendication 1, dans lequel la pompe à chaleur fournit aussi la réfrigération pour le procédé.

3. Procédé selon la revendication 1, dans lequel la réfrigération est fournie par les moyens d'un cycle réfrigérant à compression de vapeur dans lequel le milieu mis en oeuvre dans le cycle comprend un condensat obtenu en condensant partiellement le milieu compressé provenant de la pompe à chaleur avant de soumettre ledit milieu compressé à un échange de chaleur indirect avec lesdits courants séparés.

4. Procédé selon l'une quelconque des revendications précédentes, dans lequel la fonction de la pompe à chaleur est réduite en injectant une portion de gaz non condensé dans le condensat séparé avant de chauffer ce dernier par échange de chaleur indirect avec le mélange gazeux d'alimentation rentrant.

5. Procédé selon l'une quelconque des revendications précédentes, dans lequel le milieu condensé provenant de la pompe à chaleur est sous-refroidi avant la détente.

6. Procédé selon l'une quelconque des revendications précédentes, dans lequel le mélange gazeux comprend un mélange d'hydrocarbures et d'hydrogène et contient 20 à 80 moles % d'hydrogène.

7. Procédé selon l'une quelconque des revendications précédentes pour la production de gaz naturel synthétique à partir d'un courant de gaz contenant de l'hydrogène obtenu par gazéification d'un combustible solide ou au cours du raffinage de pétrole ou d'un procédé pétrochimique.

8

FIG. 1

FIG.2

HYDROGEN

FEED

SNG

0 091 830

FIG. 3